(19) **Europäisches Patentamt**

**European Patent Office**

**Office européen des brevets**

(11) Veröffentlichungsnummer: **0 332 001 B1**

(12) **EUROPÄISCHE PATENTSCHRIFT**

(45) Veröffentlichungstag der Patentschrift: **22.09.93**

(21) Anmeldenummer: **89103384.7**

(22) Anmeldetag: **27.02.89**

(51) Int. Cl.⁵: **C07C 51/43**, C07C 51/36, C07C 51/42, C11B 7/00

(54) **Verfahren zur Isolierung bzw. Reinigung von im wesentlichen methylverzweigten, gesättigten C12-C24-Fettsäuren.**

(30) Priorität: **07.03.88 DE 3807409**

(43) Veröffentlichungstag der Anmeldung:
**13.09.89 Patentblatt 89/37**

(45) Bekanntmachung des Hinweises auf die Patenterteilung:
**22.09.93 Patentblatt 93/38**

(84) Benannte Vertragsstaaten:
**DE**

(56) Entgegenhaltungen:
EP-A- 0 014 439      DE-A- 1 965 644
DE-A- 2 030 529      DE-A- 2 420 623
US-A- 2 293 676      US-A- 2 800 493
US-A- 2 812 342

THE JOURNAL OF THE AMERICAN OIL CHEMISTS' SOCIETY, Band 56, Nov. 1979, S. 823-826; D.V. KINSMAN: "Isostearic and other Branched Acids"

(73) Patentinhaber: **Henkel Kommanditgesellschaft auf Aktien**

**D-40191 Düsseldorf(DE)**

(72) Erfinder: **Struve, Alfred, Dr.**
**Am Eichelkamp 53**
**D-4010 Hilden(DE)**
Erfinder: **Baumann, Horst, Dr.**
**Vogelwarte 17**
**D-5653 Leichlingen(DE)**
Erfinder: **Schmid, Karl-Heinz, Dr.**
**Stifterstrasse 10**
**D-4020 Mettmann(DE)**
Erfinder: **Meffert, Alfred, Dr.**
**Marie-Curie-Strasse 10**
**D-4019 Monheim(DE)**

JOURNAL OF THE AMERICAN OIL CHEMISTS' SOCIETY, Band 51, 1974, S. 522-527; D.H. MCMAHON et al: "Characterization of Products from Clay Catalyzed Polymerization of Tall Oil Fatty Acids"

JOURNAL OF THE AMERICAN OIL CHEMISTS' SOCIETY, Band 62, Nr. 5, Mai 1985, S. 888-891; Y. NAKANO et al: "Thermal Alteration of Oleic Acid in the Presence of Clay Catalysts with Co-Catalysts"

**Beschreibung**

Die Erfindung betrifft ein Verfahren zur Isolierung bzw. Reinigung von im wesentlichen methylverzweigten gesättigten $C_{14}$-$C_{24}$-Fettsäuren aus technischen Fettsäuregemischen, die neben den methylverzweigten $C_{14}$-$C_{24}$-Fettsäuren gesättigte und ungesättigte, jeweils geradkettige $C_{10}$-$C_{24}$-Fettsäuren sowie methylverzweigte, ungesättigte $C_{14}$-$C_{24}$-Fettsäuren enthalten, wobei die technischen Fettsäuregemische katalytisch hydriert und die in dem hydrierten Fettsäuregemisch enthaltenen, im wesentlichen methylverzweigten, gesättigten $C_{14}$-$C_{24}$ Fettsäuren von den gesättigten, geradkettigen $C_{10}$-$C_{24}$-Fettsäuren abgetrennt und gegebenenfalls in üblicher Weise, insbesondere durch Destillation, gereinigt werden.

Methylverzweigte, gesättigte und ungesättigte $C_{10}$-$C_{24}$-Fettsäuren entstehen als Nebenprodukt bei der thermischen oder katalytischen Dimerisierung der entsprechenden ungesättigten, geradkettigen Fettsäuren. Die Struktur der bei der Dimerisierung von Tallölfettsäuren gebildeten, sogenannten Isostearinsäuren wurde beschrieben, vgl. J. Am. Oil Chem. Soc. (1979), Vol. 56, pp. 823A - 827A; ibid. (1974), Vol. 51, pp. 522 - 527; ibid. (1985), Vol. 62, 888 - 891.

Isostearinsäuren sind bei Umgebungstemperatur flüssig und infolge ihres gesättigten Charakters oxidationsunempfindlich und sind daher interessante Zwischenprodukte, insbesondere für die Herstellung von Schmierstoffen und Komponenten von kosmetischen Präparaten und Textilbehandlungsmitteln. Es sind daher mehrere Verfahren zur Isolierung und Reinigung von Isostearinsäuren aus bei der Fettsäuredimerisierung erhaltenen Fettsäuregemischen beschrieben worden. So beschreibt die US-PS 2 812 342 ein Verfahren, gemäß dem die bei der Dimerisierung von $C_{18}$-Fettsäuren anfallenden technischen Fettsäuregemische mit üblichen Nickelkatalysatoren und Wasserstoff unter Druck hydriert und die erhaltenen Isostearinsäuren gemäß einem aus der US-PS 2 293 676 bekannten Verfahren, nämlich durch Lösen in einem geeigneten Lösemittel und Auskristallisieren bei erniedrigten Temperaturen, isoliert werden.

Es wurde nun gefunden, daß man methylverzweigte gesättigte $C_{14}$-$C_{24}$-Fettsäuren unter Vermeidung der aus Gründen der Arbeitssicherheit unerwünschten Lösemitteltrennung erhalten kann, wenn man ihre Abtrennung aus des eingangs erwähnten, hydrierten technischen Fettsäuregemischen mittels einer Umnetztrennung vornimmt. Die Umnetztrennung ist ein bekanntes Verfahrensprinzip, vgl. Ullmann's Encyclopedia of Industrial Chemistry, 5. Auflage, Vol. A10, pp. 265 - 266 (1987), zur Abtrennung von Stearinsäure aus technischen Ölsäuregemischen; sie wird auch als fraktionierte Kristallisation in Anwesenheit von Netzmitteln bezeichnet.

Demgemäß ist das Verfahren der Erfindung dadurch gekennzeichnet, daß man methylverzweigte, gesättigte $C_{14}$-$C_{24}$-Fettsäuren von geradkettigen, gesättigten $C_{10}$-$C_{24}$-Fettsäuren aus hydrierten Fettsäuregemischen der eingangs genannten Art durch eine gegebenenfalls mehrstufige Umnetztrennung abtrennt.

Das Verfahren der Erfindung ist auf alle technischen Fettsäuregemische anwendbar, die methylverzweigte, gesättigte und gegebenenfalls auch methylverzweigte, ungesättigte $C_{14}$-$C_{24}$-Fettsäuren enthalten. Bevorzugt geht man von technischen Fettsäuregemischen aus, die überwiegend $C_{16}$-$C_{22}$ Fettsäuren enthalten, die bei der Dimerisierung der entsprechenden ungesättigten Fettsäuren, insbesondere von an ungesättigten $C_{18}$-Fettsäuren reichen Fettsäuregemischen, anfallen.

Die erfindungsgemäß eingesetzte Umnetztrennung stößt bei den hydrierten technischen Fettsäuregemischen, die mehr als etwa 30 Gew.-% Stearinsäure enthalten, auf Schwierigkeiten, weil sich bei diesen eine Dispersion der gesättigten, geradkettigen Fettsäuren nicht oder nur schwer ausbildet.

Gemäß einer weiteren vorteilhaften Ausführungsform der Erfindung unterwirft man daher technische Fettsäuregemische, die einen Gehalt an Stearinsäure von mehr als 30 Gew.-% nach der Hydrierung erwarten lassen, vor der katalytischen Hydrierung einer Umnetzvortrennung, um die gesättigten, geradkettigen $C_{10}$-$C_{24}$-Fettsäuren abzutrennen.

Die Hydrierung der in den eingesetzten technischen Fettsäuregemischen enthaltenen ungesättigten Fettsäuren erfolgt mit üblichen Palladium- oder Nickelkatalysatoren bei erhöhtem Druck und Temperaturen; bevorzugte Hydrierungstemperaturen liegen im Bereich von 150 bis 220°C. Im übrigen entsprechen die Hydrierungsbedingungen denen einer üblichen Fettsäurehärtung, vgl. Ullman's Encyclopedia of Industrial Chemistry, (1977) Vol. A10, pp. 267 - 269.

Gemäß einer weiteren vorteilhaften Ausführungsform der Erfindung unterwirft man zur Abtrennung der methylverzweigten, gesättigten $C_{14}$-$C_{24}$-Fettsäuren die katalytisch hydrierten Fettsäuregemische einem zweistufigen Umnetzverfahren, wobei die Trenntemperatur der zweiten Trennstufe mindestens 10°C unter derjenigen der ersten Trennstufe liegt. Dabei wird die in der ersten Stufe erhaltene methylverzweigte, gesättigte Fettsäurefraktion einer sich unmittelbar anschließenden zweiten Umnetztrennung bei tieferer Temperatur unterzogen, um zusätzliche Anteile an gesättigten, geradkettigen Fettsäuren zu entfernen.

Die Erfindung wird im folgenden anhand von bevorzugten Ausführungsbeispielen näher erläutert.

3

In den Beispielen wurde eine Monomerfettsäure eingesetzt, die als Nebenprodukt bei der Dimerisierung von Tallölfettsäure erhalten worden war.

Die eingesetzte Monomerfettsäure wies die folgenden Analysenwerte auf:

Säurezahl (SZ) 173,7

Verseifungszahl (VZ) 184,1

Jodzahl (JZ) nach Kaufmann 69,5

Hydrierjodzahl (HJZ) (mit Pd/C in Eisessig bei 20°C und ca. 1 bar Wasserstoff) 40

unverseifbare Anteile 8,0

Steigschmelzpunkt 31°C.

Das mit Pentadecansäure-methylester als innerem Standard aufgenommene Gaschromatogramm zeigte 5,5 % Palmitinsäure, 12,2 % Stearinsäure sowie 12 % "Ölsäuren" (9-Octadecensäure, Elaidinsäure und stellungsisomere Octadecensäuren) an. Die Hauptmenge der Fettsäuren bestand aus methylverzweigten Fettsäuren, die gehäuft im Peakbereich zwischen Palmitin-und Stearinsäure auftraten und nicht genau quantifizierbar sind.

Beispiel 1.

200 g der oben beschriebene Monomerfettsäure wurden mit 2 g Pd/C-Katalysator (5 % Pd auf Kohle; Engelhard-Katalysator) und 20 bar Wasserstoff bei 200°C hydriert. Das vom Katalysator abfiltrierte Hydrierungsprodukt wies eine Jodzahl von 15,2 auf.

150 g der so gehärteten Monomerfettsäure wurden im geschmolzenen Zustand bei ca. 50°C in einen sogenannten Schaber-Rührer (mit Kühlmantel versehenes, zylindrisches Gefäß aus Edelstahl mit flachem Boden und U-förmigem, dicht an die Wandung angepaßtem Rührer) zusammen mit 112,5 g einer ca. 50°C warmen Netzmittellösung (Gewichtsverhältnis von Monomerfettsäure zu Netzmittellösung 1 : 0,75) gegeben. Die Netzmittellösung bestand aus Wasser (98,7 %), 0,33 g (0,3 % Natriumdecylsulfat) und 1,12 g (1 %) Magnesiumsulfat. Durch den Kühlmantel des Schaber-Rührers wurde auf 20°C thermostatisiertes Wasser geschickt. Das Gemisch aus Fettsäure und Netzmittellösung wurde unter Rühren bei ca. 60 Umdrehungen pro Minute zur Kristallisation gebracht. Nach 40 min wurden weitere 187,5 g 20°C warme Netzmittellösung hinzugefügt. Es wurde noch 5 min bei 20°C weitergerührt. Die entstandene dünnflüssige Dispersion wurde in einer Becherzentrifuge (Fa. Heraeus Christ) bei 4000 Umdrehungen pro Minute 5 min lang zentrifugiert. Dabei erfolgte eine Auftrennung in eine leichtere, weitgehend netzmittelfreie methylverzweigte Fettsäure und eine schwerere Phase, bestehend aus der wäßrigen Netzmittellösung und den darin dispergierten kristallinen Anteilen an geradkettigen, gesättigten Fettsäuren. Nach Abtrennung der methylverzweigten Fettsäurefraktion wurde die Wasserphase mit den Fettsäurekristallen (Smp. weniger als 60°C) in einem auf 90°C erhitzten Scheiderohr aufgetrennt. Die aufgetrennten Anteile an flüssigen und festen Fettsäuren verhielten sich gewichtsmäßig wie 69,5 : 31,5. Der Festanteil wies eine Jodzahl von 2 und einen Schmelzpunkt von 46 bis 48°C, der Flüssiganteil eine Jodzahl von 19,3 und einen Trübungspunkt von 16°C auf.

Beispiel 2.

An der vorstehend beschriebenen Monomerfettsäure wurde die Verfahrensvariante mit Umnetzvortrennung wie folgt demonstriert:

40 kg geschmolzene Monomerfettsäure wurden in einem Kessel mit eng an die Wandung angepaßtem Gitterrührer zusammen mit 30 kg der im Beispiel 1 beschriebenen Netzmittellösung (Gewichtsverhältnis 1 : 0,75) unter Rühren bei ca. 60 Umdrehungen pro Minute von ca. 50°C auf 20 bis 22°C heruntergekühlt, und zwar durch Beschickung des Kühlmantels des Kessels mit 19 bis 20°C warmem Wasser.

Anschließend wurden unter fortgesetztem Rühren weitere 50 kg der auf 20°C temperierten Netzmittellösung hinzugegeben (Gewichtsverhältnis Fettsäure : Netzmittellösung insgesamt 1 : 2). Nach weiterem ca. 20-minütigem Rühren wurde die entstandene dünnflüssige Dispersion mit einer Vollmantelzentrifuge getrennt. Es erfolge Auftrennung in eine leichtere, mit wenig Netzmittellösung (ca. 5 %) austretende flüssige Fettsäure und in eine schwerere Phase aus Netzmittellösung und darin dispergierten Fettsäurekristallen. Die gewichtsmäßige Fettsäureaufteilung betrug 85 (flüssig) : 15 (fest).

Nach Beendigung des Trennvorgangs in der Zentrifuge wurden beide Fraktionen auf ca. 80°C erhitzt. Die separierenden Netzmittellösungen wurden abgelassen; die Fettsäuren wurden zweimal mit Wasser bei 80°C gewaschen. Die flüssige Fraktion wurde im Vakuum (ca. 20 mbar) bei 100°C unter Rühren getrocknet und mit 2 % eines üblichen Nickelkatalysators (enthaltend ca. 25 % Nickel auf Kieselgur) bei 200°C mit 20 bar Wasserstoff hydriert. Das vom Katalysator abfiltrierte Produkt wies eine Jodzahl von 28 und einen Steigschmelzpunkt von 36°C auf.

25 kg des gehärteten Produktes wurden erneut in der oben beschriebenen Weise, d.h. bei 20°C und mit dem gleichen Verhältnis von Fettsäure zu Netzmittellösung, umgenetzt, dispergiert und getrennt. Es ergab sich eine Auftrennung von flüssiger zu fester Fettsäure im Gewichtsverhältnis von 74 : 26.

Die in der vorstehenden Stufe erhaltene flüssige Fettsäure wurde unmittelbar im netzmittelfeuchten Zustand einer weiteren Umnetztrennung unterworfen, wobei die Abkühlung bei ca. 40°C begann und bei 10°C endete. Alle übrigen Maßnahmen wurden wie oben beschrieben getroffen.

Nunmehr ergab sich eine Auftrennung in der Zentrifuge im Verhältnis von 89 (flüssig) : 11 (fest).

Nach Beendigung der Trennung wurde der flüssige Anteil wie oben im Anschluß an die erste Trennstufe beschrieben, gereinigt und getrocknet.

Der Flüssiganteil aus der letzten Trennstufe (89 %) wurde im Vakuum bei 0,5 mbar destilliert. Man erhielt:

9 % Vorlauf (Sdp. 50 bis 150°C/0,5 mbar)

82 % Hauptlauf (Sdp. 150 bis 165°C/0,5 mbar)

9,5 % Rückstand.

Für die methylverzweigten, gesättigten Fettsäuren ergab sich als Endausbeute 56 % (vor der Destillation) bzw. 46 % (Hauptlauf der Destillation).

In der nachfolgenden Tabelle sind die Analysen von Produkten der verschiedenen Trennstufen zusammengefaßt.

Tabelle

| Analyse | 1. Trennung 85 : 15 | | 2. Trennung[1] 74 : 26 | | 3. Trennung 89 : 11 | | Destillation 82[2] : 9[3] | |
|---|---|---|---|---|---|---|---|---|
| Auftrennung (Gew.-Teile; flüssig : fest) | flüssig | fest | flüssig | fest | flüssig | fest | flüssig | fest |
| SZ | 170 | 192 | 160 | 174 | 161 | 166 | 172 | 99 |
| VZ | 181 | 196 | 176 | 186 | 175 | 184 | 183 | 113 |
| JZ | 78 | 24 | 33 | 16 | 34 | 22 | 25 | 92 |
| HJZ | 42 | 17 | <2 | <2 | <2 | <2 | <2 | – |
| Trübungspunkt (°C) | 17 | – | 12 | – | 6 | – | 8 | – |
| Smp. (°C) | – | 43-9 | – | 40-3 | – | 30-40 | – | – |
| $C_{16}$ ges.[4] | 4,4 | 11 | 3,9 | 7 | 3,5 | 10 | 4,1 | 2,6 |
| $C_{18}$ ges.[5] | 3,8 | 51 | 4,1 | 40 | 3,2 | 16 | 4,8 | 0,8 |

1) mit gehärtetem Produkt (JZ 28)
2) Hauptlauf
3) Vorlauf
4) Palmitinsäure
5) Stearinsäure

Beispiel 3

Die Anwendung des erfindungsgemäßen Verfahrens nach Art des Beispiels 1 führte bei der folgenden Monomerfettsäure wegen des hohen Gehalts an geradkettigen Verbindungen (> 30 %) nicht zu einer Dispersion, sondern zur Bildung grober Zusammenballungen:
St. 171,9
VZ. 182,0
JZ. 77,8
HJZ. 38,0
1,8 % Palmitinsäure
12,5 % Stearinsäure
19,3 % "Ölsäuren"

Hier war daher eine Vortrennung notwendig. D.h. diese Monomerfettsäure wurde gemäß den unter Beispiel 2/1. Trennstufe beschriebenen Bedingungen einer Umnetztrennung unterworfen. Dabei wurde eine Auftrennung um 13,4 % Festanteil mit JZ. 34,5 und 86,6 % Flüssiganteil mit Jodzahl 85,9 erreicht. Der Festanteil bestand zu über 50 % aus Stearinsäure. Im Flüssiganteil lag die Summe an geradkettigen Fettsäuren jetzt deutlich unter 30 %. Damit war die Voraussetzung für die Anwendung des im Beispiel 1 beschrittenen Weges gegeben.

**Patentansprüche**

1. Verfahren zur Isolierung bzw. Reinigung von im wesentlichen methylverzweigten, gesättigten $C_{14}$-$C_{24}$-Fettsäuren aus technischen Fettsäuregemischen, die neben den methylverzweigten $C_{14}$-$C_{24}$-Fettsäuren gesättigte und ungesättigte, jeweils geradkettige $C_{10}$-$C_{24}$-Fettsäuren sowie methylverzweigte ungesättigte $C_{14}$-$C_{24}$-Fettsäuren enthalten, wobei die technischen Fettsäuregemische katalytisch hydriert und die in dem hydrierten Fettsäuregemisch enthaltenen, im wesentlichen methylverzweigten, gesättigten $C_{14}$-$C_{24}$ Fettsäuren von den gesättigten, geradkettigen $C_{10}$-$C_{24}$-Fettsäuren abgetrennt und gegebenenfalls in üblicher Weise, insbesondere durch Destillation, gereinigt werden, dadurch gekennzeichnet, daß man die methylverzweigten, gesättigten $C_{14}$-$C_{24}$-Fettsäuren von den geradkettigen, gesättigten $C_{10}$-$C_{24}$-Fettsäuren durch eine gegebenenfalls mehrstufige Umnetztrennung abtrennt.

2. Verfahren nach Anspruch 1, dadurch gekennzeichnet, daß man von technischen Fettsäuregemischen ausgeht, die überwiegend $C_{16}$-$C_{22}$-Fettsäuren enthalten.

3. Verfahren nach Anspruch 1 oder 2, dadurch gekennzeichnet, daß man technische Fettsäuregemische, die einen Gehalt an Stearinsäure von mehr als 30 Gew.-% nach der Hydrierung erwarten lassen, zur Abtrennung der gesättigten, geradkettigen $C_{10}$-$C_{24}$-Fettsäuren vor der katalytischen Hydrierung einer Umnetzvortrennung unterwirft.

4. Verfahren nach mindestens einem der Ansprüche 1 bis 3, dadurch gekennzeichnet, daß man zur Abtrennung der methylverzweigten, gesättigten $C_{14}$-$C_{24}$-Fettsäuren die katalytisch hydrierten Fettsäuregemische einer zweistufigen Umnetztrennung unterwirft, wobei die Trenntemperatur der zweiten Trennstufe mindestens 10°C unter derjenigen der ersten Trennstufe liegt.

5. Verfahren nach mindestens einem der Ansprüche 1 bis 3, dadurch gekennziechnet, daß man die Hydrierung der ungesättigten Fettsäuren in Gegenwart von Nickel- oder Palladiumkatalysatoren bei Überdruck und bei Temperaturen von 150 bis 220°C vornimmt.

**Claims**

1. A process for isolating or purifying substantially methyl-branched saturated $C_{12-24}$ fatty acids from technical fatty acid mixtures which, in addition to the methyl-branched $C_{14-24}$ fatty acids, contain saturated and unsaturated, straight-chain $C_{10-24}$ fatty acids and methyl-branched unsaturated $C_{14-24}$ fatty acids, the technical fatty acids being catalytically hydrogenated and the substantially methyl-branched saturated $C_{14-24}$ fatty acids present in the hydrogenated fatty acid mixture being removed and optionally purified by standard methods, more particularly by distillation, characterized in that the methyl-branched saturated $C_{14-24}$ fatty acids are removed from the straight-chain saturated $C_{10-24}$ fatty acids by an optionally multi-stage rolling-up separation.

2. A process as claimed in claim 1, characterized in that technical fatty acid mixtures predominantly containing $C_{16-22}$ fatty acids are used as the starting material.

3. A process as claimed in claim 1 or 2, characterized in that technical fatty acid mixtures which are expected to contain more than 30% by weight stearic acid after hydrogenation are subjected to preliminary rolling-up separation before catalytic hydrogenation to remove the saturated straight-chain $C_{10-24}$ fatty acids.

4. A process as claimed in at least one of claims 1 to 3, characterized in that, to remove the methyl-branched saturated $C_{14-24}$ fatty acids, the catalytically hydrogenated fatty acid mixtures are subjected to a two-stage rolling-up separation, the temperature of the second separation stage being at least 10 ° C below that of the first separation stage.

5. A process as claimed in at least one of claims 1 to 3, characterized in that the hydrogenation of the unsaturated fatty acids is carried out in the presence of nickel or palladium catalysts under excess pressure and at temperatures of 150 to 220 ° C.

**Revendications**

1. Procédé d'isolement ou de purification d'acides gras en $C_{14}$ à $C_{24}$ saturés, ramifiés par un méthyle essentiellement à partir de mélanges d'acides gras industriels qui renferment à côté des acides gras en $C_{14}$ à $C_{24}$ ramifiés par un méthyle, des acides gras en $C_{10}$ à $C_{24}$ saturés et insaturés, respectivement à chaîne droite ainsi que des acides gras en $C_{14}$ à $C_{24}$ non saturés, ramifiés par un méthyle, procédé dans lequel les mélanges industriels d'acides gras sont hydrogénés catalytiquement et les acides gras en $C_{14}$ à $C_{24}$ saturés, essentiellement ramifiés par un méthyle contenus dans le mélange d'acides gras hydrogénés sont séparés des acides gras en $C_{10}$ à $C_{24}$ à chaîne droite saturés et le cas échéant sont purifiés d'une manière habituelle, en particulier par distillation, caractérisé en ce que l'on sépare les acides gras en $C_{14}$ à $C_{24}$ saturés, ramifiés par un méthyle, des acides gras en $C_{10}$ à $C_{24}$ saturés à chaîne droite par une séparation par contact humide, éventuellement en plusieurs étages.

2. Procédé selon la revendication 1, caractérisé en ce que l'on part de mélanges industriels d'acides gras qui contiennent principalement des acides gras en $C_{16}$ à $C_{22}$.

3. Procédé selon la revendication 1 ou 2, caractérisé en ce que l'on soumet les mélanges industriels d'acides gras qui laissent espérer une teneur en acide stéarique de plus de 30 % en poids après hydrogénation, en vue de la séparation des acides gras en $C_{12}$ à $C_{20}$ en chaîne droite, saturés avant l'hydrogénation catalytique à une séparation par contact humide.

4. Procédé selon au moins une des revendications 1 à 3, caractérisé en ce que l'on soumet en vue de la séparation des acides gras en $C_{14}$ à $C_{24}$ saturés,ramifiés par un méthyle, les mélanges d'acides gras hydrogénés catalytiquement, à une séparation par contact humide en deux étapes, dans laquelle la température de séparation de la deuxième étape de séparation, se situe au moins 10 ° C en dessous de celle de la première étape de séparation.

5. Procédé selon au moins une des revendications 1 à 3, caractérisé en ce que l'on entreprend l'hydrogénation des acides gras non saturés en présence de catalyseurs à base de nickel ou de palladium avec une surpression et à des températures de 150 à 220 ° C.